# FASCICULE DE BREVET EUROPEEN

(11) **EP 2 513 045 B1**
(45) Date de publication et mention de la délivrance du brevet: **07.11.2018**
(21) Numéro de dépôt: 10776759.2
(22) Date de dépôt: 29.09.2010
(51) Int. Cl.: C07C 227/42, C07C 67/52, C07C 69/157, C07C 229/26, C07C 69/86, C07C 67/28

(54) **PROCEDE DE PREPARATION D'UN SEL D'ACIDE O- ACETYLSALICYLIQUE ET D'UN ACIDE AMINE BASIQUE**
VERFAHREN ZUR HERSTELLUNG EINES SALZES AUS O-ACETYLSALICYLSÄURE UND EINER BASISCHEN AMINOSÄURE
METHOD FOR PREPARING A SALT OF O-ACETYLSALICYLIC ACID AND A BASIC AMINO ACID

(30) Priorité: 16.06.2010 FR 1002539; 30.09.2009 FR 0904653
(43) Date de publication de la demande: 24.10.2012
(73) Titulaire: M2I DEVELOPMENT, 64170 Lacq (FR)
(72) Inventeur: RIVIERE, Philippe, 31500 Toulouse (FR); REULET, Philippe, 75015 Paris (FR); LEBORGNE, Fabrice, 81000 Albi (FR); GUYOT, Daniel, 31380 Montjoire (FR)
(74) Mandataire: Regimbeau
(86) Numéro de dépôt international: PCT/FR2010/000646
(87) Numéro de publication internationale: WO 2011/039432

(56) Documents cités:
- EP-B1- 1 365 737
- WO-A2-2006/128600
- FR-A1- 2 115 060

## Description

La présente invention concerne un procédé de préparation d'un sel d'acide *O*-acétylsalicylique et d'un acide aminé basique.

L'action analgésique de l'acide *O*-acétylsalicylique (Aspirine ®) est bien connue. Ce composé a des applications multiples dans le domaine de la santé, non seulement comme analgésique mais également comme antipyrétique et anti-rhumatisme, sans que cette liste soit limitative. L'une des difficultés constatées pour l'utilisation de l'acide *O*-acétylsalicylique est sa faible solubilité dans l'eau. Il a néanmoins été proposé (JP-A-55127345) de remédier à cet inconvénient en utilisant un sel de l'acide *O-*acétylsalicylique avec un acide aminé basique, notamment la L-lysine, la D,L-lysine ou l'arginine. Ces sels ont une bonne solubilité dans l'eau, ce qui permet d'envisager des applications parentérales de l'acide *O-*acétylsalicylique.

Pour obtenir un tel sel hydrosoluble ayant une bonne stabilité, on a déjà proposé dans le brevet européen 1 365 737 un procédé de préparation, mais ce procédé présente un certain nombre d'inconvénients : il est en effet d'une exécution lente en raison des précautions qu'il faut prendre pour réduire la désacétylation de l'acide *O*-acétylsalicylique lorsqu'on le fait réagir avec l'acide aminé qui lui confère la solubilité recherchée ; par ailleurs, dans ce procédé, lorsque la réaction produisant le sel désiré est accomplie, on fait précipiter ledit sel dans des conditions de faible agitation, ce qui entraine un temps de précipitation important ; enfin, on est amené à laver le précipité obtenu avec un solvant organique avant de le sécher, de façon à éliminer le plus d'eau possible, ce qui améliore la stabilité du produit obtenu et cette étape lavage/séchage augmente encore le temps nécessaire pour la production du sel. Il en résulte que le prix de revient de la composition pulvérulente obtenue est élevé et qu'il est donc souhaitable de réduire le temps de fabrication pour améliorer la productivité et faire baisser le prix de revient de la composition pulvérulente obtenue, ce qui est le but de l'invention.

Dans le procédé selon l'invention, on réalise la réaction de l'acide aminé sur l'acide *O*-acétylsalicylique avec un léger défaut de l'acide aminé basique par rapport à la stoechiométrie de sorte que le milieu est légèrement acide ; on sait qu'un tel pH favorise l'apparition de salicylates mais l'on adopte des conditions réactionnelles telles que la réaction s'effectue très rapidement : on préconise l'utilisation de solutions relativement concentrées des réactifs à une température relativement basse, car, de la sorte, la salification s'effectue rapidement alors que l'apparition des salicylates est extrêmement limitée ; cette utilisation de solutions à fortes concentrations permet de réduire considérablement le temps de réaction. Le milieu réactionnel est transféré en fin de réaction dans un cristalliseur où on lui additionne de l'acétone, le milieu de précipitation dans le cristalliseur étant maintenu sous une agitation énergique pour réduire autant que possible le temps nécessaire à la cristallisation. Au contraire, dans le procédé du brevet européen 1 365 737, l'agitation dans le cristalliseur est très faible pour obtenir le type de cristallisation qui est recherché et, en conséquence, le temps de cristallisation est très long. Enfin, dans le procédé antérieurement décrit, le séchage s'effectue obligatoirement après un lavage avec un solvant organique de façon à évacuer tous les liquides et réactifs en excès du milieu de précipitation, après quoi, le produit est séché sous pression réduite. Selon l'invention, on a constaté qu'un tel lavage avant séchage n'était pas toujours nécessaire.

La mise en oeuvre du procédé selon l'invention peut s'effectuer sans difficulté sur une installation fonctionnant en continu, la sortie du cristalliseur étant amenée sur un filtre. Dans une première variante, ce filtre peut être un filtre-bande comportant, sous la toile du filtre, un caisson à pression réduite qui accélère la séparation des liquides par rapport aux grains de poudre obtenus, la partie finale du filtre-bande étant soumise à un flux d'air pulsé. Dans une autre variante de l'installation mettant en oeuvre le procédé selon l'invention, la sortie du cristalliseur est amenée sur un filtre-sécheur rotatif sous pression.

La présente invention a, en conséquence, pour objet un procédé de préparation d'un sel d'acide *O*-acétylsalicylique d'un acide aminé basique dans lequel :
- on réunit dans un réacteur, d'une part, une solution aqueuse d'un acide aminé basique et, d'autre part, une solution d'acide *O*-acétylsalicylique dans un solvant organique miscible à l'eau, à une température inférieure à 30°C sous pression normale ;
- on laisse la réaction se produire pendant un temps suffisant pour obtenir le sel désiré ;
- dans un cristalliseur, on ajoute au milieu réactionnel ainsi constitué, à une température inférieure à 40°C et sous une pression normale, de l'acétone pour faire précipiter le sel désiré ;
- on isole du milieu liquide le produit obtenu et on le sèche à une température inférieure à 50°C ;
caractérisé en ce que l'acide aminé est introduit dans le réacteur en une quantité telle qu'il soit présent dans le milieu réactionnel en léger défaut par rapport à la stoechiométrie, la concentration pondérale des solutions des deux réactifs étant inférieure à la saturation mais supérieure à 5% en poids pour l'acide *O*-acétylsalicylique et à 20% en poids pour l'acide aminé, la réaction s'effectuant en suspension, à une température choisie entre -15°C et +30°C en fonction du solvant utilisé et sous agitation, et que le sel, suite à l'addition d'acétone dans le cristalliseur, précipite sous une agitation énergique, ledit sel étant séché après qu'il ait été isolé de son milieu de précipiation, en ce que le rapport molaire acide O-acétylsalicylique/acide aminé basique des réactifs introduits dans le réacteur est compris entre 1 :0,97 et 1 :0,70 bornes incluses, en ce que l'agitation dans le cristalliseur est effectuée avec une dépense d'énergie comprise entre 0, 5 et 2W par litre de milieu et en ce que le procédé est mené en continu.

Le procédé selon l'invention peut être mis en oeuvre en choisissant comme acide aminé basique la lysine, l'arginine ou l'ornithine ; on préfère que la mise eu oeuvre ait lieu avec la lysine quelque soit sa forme, racémique ou non, monohydrate ou non. Le solvant peut être choisi dans le groupe formé par les alcools inférieurs tels que le méthanol, l'éthanol et l'isopropanol, les éthers comme le tétrahydrofurane, les cétones comme l'acétone ou leurs mélanges. On préfère l'éthanol et l'acétone.

Le procédé est mené en continu ; on peut prévoir que le temps de réaction dans le réacteur soit inférieur à 1 heure ; on peut prévoir également que la température dans le cristalliseur soit choisie entre -15°C et 15°C (bornes incluses); le temps entre l'entrée du milieu de réaction dans le cristalliseur et l'isolement du sel précipité par rapport à la phase liquide du milieu de précipitation est avantageusement inférieur à 1 heure. Dans une première variante du procédé selon l'invention, le solvant organique utilisé pour la mise en solution de l'acide *O*-acétylsalicylique est l'éthanol et la réaction dudit acide avec l'acide aminé basique est effectuée à une température comprise entre -15°C et 0°C (bornes incluses). Dans une deuxième variante, le solvant organique utilisé pour la mise en solution de l'acide *O*-acétylsalicylique est l'acétone et la réaction dudit acide avec l'acide aminé basique est effectuée à une température comprise entre -5°C et +30°C (bornes incluses). On a constaté que la mise en oeuvre du procédé selon l'invention permettait d'obtenir un produit dont le taux en salicylates était inférieur à 0,2%, ce produit présentant une très bonne stabilité.

Dans un premier mode de mise en oeuvre du procédé selon l'invention, le séchage du sel précipité dans le cristalliseur est effectué sur un filtre-sécheur à bande, dont une face est soumise à une pression réduite, la partie du filtre qui reçoit le produit assurant sa séparation primaire par rapport au milieu de précipitation et la partie suivante assurant un séchage par air pulsé à une température inférieure à 50°C pendant un temps compris entre 0,1 et 2 h. Dans un second mode de mise en oeuvre du procédé selon l'invention, le séchage du sel précipité dans le cristalliseur est effectué en envoyant la suspension produite dans le cristalliseur dans un filtre-sécheur rotatif sous pression d'air, l'élément de filtration étant porté par un organe rotatif, qui est entrainé autour de l'axe du stator du filtre-sécheur, le liquide filtré à partir de la suspension sortant selon l'axe du stator après avoir traversé l'élément de filtration, l'alimentation en suspension s'effectuant sur un premier secteur du stator, le séchage par soufflage d'air du solide arrêté par l'élément de filtration étant effectué dans un second secteur du stator atteint par l'organe rotatif après le premier secteur dans le sens de la rotation du rotor, le solide retenu à la surface de l'élément de filtration étant raclé dans un troisième secteur du stator, postérieur au second dans le sens de rotation du rotor et étant évacué en continu hors du filtre-sécheur. La présente description se réfère aussi à une composition pulvérulente obtenue par le procédé ci-dessus défini, dans laquelle plus de 90% des grains de la composition ont un diamètre moyen compris entre 5 et 150µm.

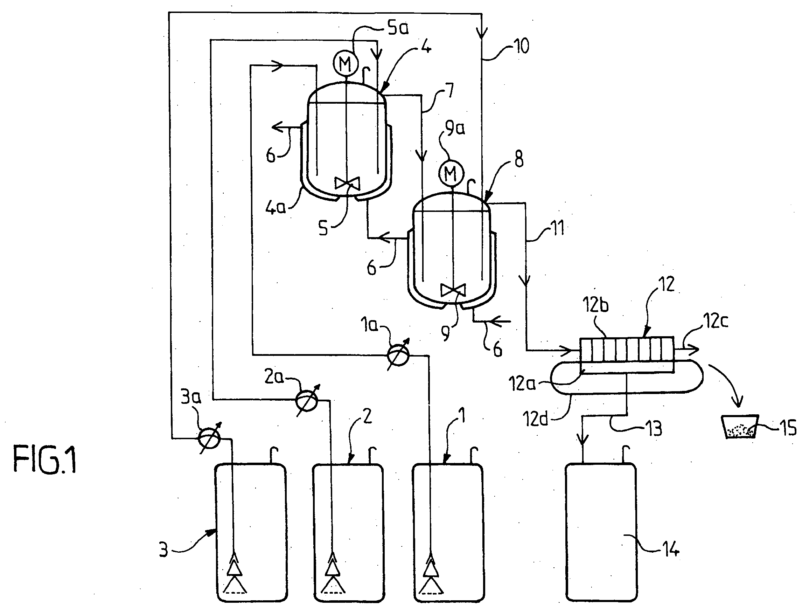

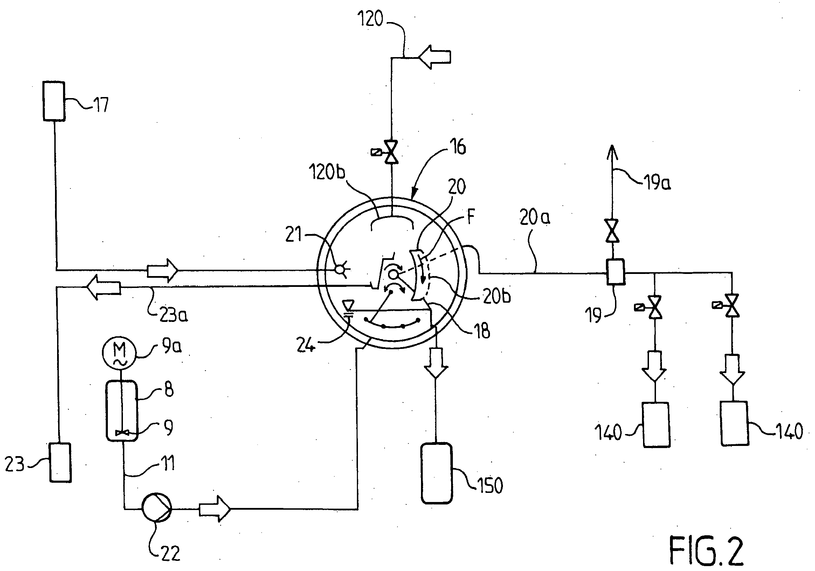

Pour mieux faire comprendre l'objet de l'invention, on va décrire maintenant schématiquement une installation permettant l'exécution en continu du procédé selon l'invention, qu'il s'agisse du premier mode de mise en oeuvre comportant un filtre-sécheur à bande (figure 1) ou du deuxième mode de mise en oeuvre comportant un filtre-sécheur rotatif sous pression (figure 2).

En se référant à la figure 1, on voit que l'on a désigné par 1, 2 et 3 les réservoirs dans lesquels sont stockées les matières premières mises en oeuvre, à savoir, respectivement, en 1, une solution d'acide *O-*acétylsalicylique dans un solvant organique, en 2, une solution aqueuse de D,L-lysine et en 3, de l'acétone. Le réservoir 1 contient une solution d'acide *O*-acétylsalicylique dans l'éthanol ou l'acétone. Les pompes 1a, 2a, 3a prélèvent dans les réservoirs 1, 2, 3 respectivement, les flux liquides qui sont nécessaires à la mise en oeuvre en continu du procédé selon l'invention.

L'installation comprend un réacteur 4 équipé d'un agitateur 5 entrainé par un moteur 5a. Le réacteur 4 comporte une chemise de refroidissement 4a, qui permet de maintenir le liquide à l'intérieur du réacteur 4 à la température choisie grâce à une circulation de liquide caloporteur. Le réacteur 4 est alimenté en continu par les pompes 1a et 2a qui envoient les flux liquides correspondants au fond du réacteur 4. La sortie du réacteur 4 s'effectue par débordement dans la canalisation de sortie 7 qui alimente un cristalliseur 8.

Dans le cristalliseur 8 se trouve un agitateur 9 entrainé par un moteur 9a. Le cristalliseur 8 est alimenté par la pompe 3a grâce à une canalisation 10 ; il est équipé d'une chemise de refroidissement qui permet de maintenir le milieu intérieur à une température choisie dans l'intervalle (-15°C, +15°C) (bornes incluses). Le cristalliseur 8 a une sortie par débordement, qui s'effectue grâce à une canalisation 11; cette sortie est envoyée sur un filtre sécheur à bande 12, dont la partie inférieure comporte un caisson 12a sous pression réduite et dont la partie supérieure est alimentée, au travers d'un injecteur 12b, par un débit de 15 m³/h, d'un air à 25°C. La bande roulante 12d du filtre sécheur 12 a une largeur de 0,575 m et une longueur active de 2,8 m. Le caisson 12a est relié par une canalisation 13 à une cuve 14, qui récupère les solvants mis en oeuvre dans le procédé.

Dans une première variante de ce premier mode de mise en oeuvre du procédé selon l'invention, l'acide *O*-acétylsalicylique est dissout dans l'éthanol pour former une solution à 8 % en poids (densité 0,83) et la solution aqueuse de D,L-lysine est à 27% en poids (densité 1,07). Le débit de la pompe la est de 0,46 1/min ; le débit de la pompe 2a est de 0,083 l/min ; le volume du réacteur 4 est de 15 1. La température du liquide à l'intérieur du réacteur 4 est (-5°C±2°C) Le temps de contact des deux réactifs dans le réacteur 4 est estimé à 30±3 min. L'énergie d'agitation fournie par le moteur 5a est de 0,5 W/litre de milieu. Le réacteur 4 se vide par débordement en continu dans le cristalliseur 8 par la canalisation 7 à un débit de 0,55 l/min ; le cristalliseur 8 a un volume interne de 60L ; l'acétone provenant du réservoir 3 est délivrée par la canalisation 10 à un débit de 0,56 l/min. L'énergie fournie par le moteur 9a pour assurer l'agitation dans le cristalliseur 8 est de 0,5 W par litre de milieu. Le temps de séjour du milieu de précipitation dans le cristalliseur 8 est estimé à 55±5min ; la température dans le cristalliseur 8 est maintenue à (-5°C±2°C). Le filtre-sécheur 12 est alimenté par débordement à partir du cristalliseur 8. La pression dans le caisson 12a est comprise entre 10 et 100 mbars. Le débit d'air envoyé par l'injecteur 12b est de 15 m³/h, l'air ayant une température de 25°C. La vitesse de la bande filtrante 12d est de 2,5 m/h. Le produit 15 obtenu est une poudre blanche, dont 90% des grains ont un diamètre moyen compris entre 5 et 150 microns : la production est de 3,3 kg/h.

Dans une deuxième variante du premier mode de mise en oeuvre du procédé selon l'invention, on utilise le même type d'appareillage que celui précédemment décrit pour la première variante dudit premier mode de mise en oeuvre avec des volumes de réacteur inférieurs. L'installation comprend un réacteur 4 équipé d'un agitateur 5 entrainé par un moteur 5a avec une consommation d'énergie de 0,7 W par litre de milieu. Le réacteur 4 a un volume interne de 1,5 1 et comporte une chemise de refroidissement 4a, qui permet de maintenir le liquide à l'intérieur du réacteur 4 à une température que l'on fixe à 25°C±2°C grâce à une circulation de liquide caloporteur. Le réacteur 4 est alimenté en continu par les pompes 1a et 2a, qui envoient les flux liquides correspondants au fond du réacteur 4. La sortie du réacteur 4 s'effectue par débordement dans la canalisation de sortie 7 qui alimente un cristalliseur 8 ayant un volume interne de 6 1. Le solvant de l'acide *O-*acétylsalicylique est l'acétone et la concentration de la solution est de 17,7% en poids (densité 0,86). La concentration de la solution aqueuse de lysine est de 33% en poids (densité 1,09). Le débit de la pompe 1a est de 0,224 l/min ; le débit de la pompe 2a est de 0,068 l/min. Ceci correspond à un rapport molaire (acide O-acétylsalicylique/acide aminé basique) de 1:0,89. Le temps de séjour du milieu de salification dans le réacteur 4 est estimé à 4,5±0,5 min. L'alimentation en acétone du cristalliseur 8 est fournie par la pompe 3a à raison de 0,3 l/min ; l'énergie d'agitation est de 0,7W par litre de milieu ; la température dans le cristalliseur est maintenue à 11°C±2°C ; le temps de contact pour la précipitation est estimé à 9,5±0,5 min. Le fonctionnement du filtre sécheur est le même que pour la première variante. La poudre 15 obtenue a plus de 90% de ses grains, qui ont un diamètre moyen compris entre 5 et 150 microns : la production est de 3,2 kg/heure. On a analysé le produit obtenu et on a constaté qu'il contenait 0,06 % en poids de salicylates.

Dans l'une ou l'autre des deux variantes du premier mode de mise en oeuvreci-dessus décrit, on peut utiliser, pour réaliser le deuxième mode de mise en oeuvre du procédé selon l'invention, un autre matériel de filtration/séchage que celui précédemment décrit, de façon à réduire encore le temps de fabrication. Pour ce faire, on utilise un filtre-sécheur rotatif sous pression. La figure 2 représente un tel filtre-sécheur désigné par 16 dans son ensemble et utilisé en aval du cristalliseur 8. Les organes qui ont, dans la figure 2, la même fonction que des organes équivalents de la figure 1 ont été désignés sur la figure 2 par les mêmes chiffres de référence que sur la figure 1.

Le cristalliseur 8 est muni d'un agitateur 9 entraîné par un moteur 9a; il alimente la canalisation 11, qui, par la pompe 22, envoie sous pression dans le filtre-sécheur 16 la suspension obtenue dans le cristalliseur 8.

Le filtre-sécheur 16 a la forme d'un stator cylindrique plat associé à un rotor, dont l'axe est solidaire d'un organe rotatif 20; dans la réalisation décrite et représentée sur la figure 2, l'organe rotatif 20 est un sabot, qui porte l'élément de filtration 20b et qui, dans sa rotation (0,15 à 1,5 tours/min dans le sens de la flèche F), vient, dans un premier secteur, en vis-à-vis de l'injection de suspension obtenue grâce à la pompe 22. La fraction liquide de la suspension est évacuée par l'axe de rotation du sabot 20 vers une canalisation 20a. Dans sa rotation, le sabot 20 passe éventuellement devant une tête de lavage 21 alimentée en acétone par le réservoir 17 ; l'excès d'acétone est récupéré par une canalisation 23a, qui alimente un récipient de recueil 23. L'exécution d'un tel lavage éventuel n'entraine aucune augmentation du temps total de fabrication; en effet après être passé au cours de sa rotation devant la tête de lavage 21, le sabot 20 vient dans un deuxième secteur en vis-à-vis d'une cloche de soufflage d'air 120b alimentée par la canalisation 120 en air sec à 25°C ; puis le sabot 20 poursuit sa rotation, vers un troisième secteur, où il est gratté extérieurement par un racleur 18, et revient progressivement au droit du secteur de l'injection de la suspension. La poudre sèche raclée est envoyée vers un récipient 150, qui collecte ainsi le produit obtenu par le procédé. L'acétone filtrée par le sabot 20 et transportée par la canalisation 20a est envoyée vers des cuves de récupération 140. L'appareil fonctionne sous pression pour que le filtrage s'effectue rapidement et l'air comprimé introduit dans l'appareil est détendu par le détendeur 19 et libéré à l'extérieur par la canalisation 19a.

Un tel filtre rotatif est notamment vendu par la société allemande dite « KMPT AG. » et permet d'obtenir les mêmes performances de production, que celles atteintes avec un filtre-bande (12) et détaillées dans les deux modes de mise en oeuvre précédemment décrits. L'organe rotatif d'un filtre de ce type pourrait aussi être un tambour perforé portant l'élément de filtration.

## Revendications

1. Procédé de préparation d'un sel d'acide *O*-acétylsalicylique d'un acide aminé basique, dans lequel :
- on réunit dans un réacteur (4), d'une part, une solution aqueuse d'un acide aminé basique et, d'autre part, une solution d'acide *O*-acétylsalicylique dans un solvant organique miscible à l'eau, à une température inférieure à 30°C sous pression normale ;
- on laisse la réaction se produire pendant un temps suffisant pour obtenir le sel désiré ;
- dans un cristalliseur (8), on ajoute au milieu réactionnel ainsi constitué, à une température inférieure à 40°C et sous une pression normale, de l'acétone pour faire précipiter le sel désiré ;
- on isole le produit du milieu liquide obtenu et on le sèche à une température inférieure à 50°C ;
**caractérisé en ce que** l'acide aminé est introduit dans le réacteur en une quantité telle qu'il soit présent dans le milieu réactionnel en léger défaut par rapport à la stoechiométrie, la concentration pondérale des solutions des deux réactifs étant inférieure à la saturation mais supérieure à 5% en poids pour l'acide *O*-acétylsalicylique et à 20 % en poids pour l'acide aminé, la réaction s'effectuant en suspension à une température choisie entre -15°C et +30°C en fonction du solvant utilisé et sous agitation, et que le sel, suite à l'addition d'acétone dans le cristalliseur, précipite sous une agitation énergique, ledit sel étant séché après qu'il ait été isolé de son milieu de précipitation, **en ce que** le rapport molaire acide *O*-acétylsalicylique/acide aminé basique des réactifs introduits dans le réacteur (4) est compris entre 1 :0,97 et 1 :0,70 bornes incluses, **en ce que** l'agitation dans le cristalliseur (8) est effectuée avec une dépense d'énergie comprise entre 0,5 et 2W par litre de milieu et **en ce que** le procédé est mené en continu.

2. Procédé selon la revendication 1, **caractérisé en ce que** l'acide aminé basique est la lysine ou l'arginine ou l'ornithine.

3. Procédé selon la revendication 2, **caractérisé en ce que** l'acide aminé basique est la lysine quelle que soit sa forme, racémique ou non, monohydrate ou non.

4. Procédé selon l'une des revendications 1 à 3, **caractérisé en ce que** le temps de réaction dans le réacteur (4) est inférieur à 1 heure.

5. Procédé selon l'une des revendications 1 à 4, **caractérisé en ce que** la température dans le cristalliseur (8) est choisie entre -15°C et +15°C bornes incluses.

6. Procédé selon l'une des revendications 1 à 5 **caractérisé en ce que** le temps entre l'entrée du milieu de réaction dans le cristalliseur (8) et l'isolement du sel précipité par rapport à la phase liquide du milieu de précipitation, est inférieur à 1 heure.

7. Procédé selon l'une des revendications 1 à 6, **caractérisé en ce que** le solvant organique utilisé pour la mise en solution de l'acide *O*-acétylsalicylique est l'éthanol et la réaction dudit acide avec l'acide aminé basique est effectuée à une température comprise entre -15°C et 0°C bornes incluses.

8. Procédé selon l'une des revendications 1 à 6, **caractérisé en ce que** le solvant organique utilisé pour la mise en solution de l'acide *O*-acétylsalicylique est l'acétone et la réaction dudit acide avec l'acide aminé basique est effectuée à une température comprise entre -5°C et +30°C bornes incluses.

9. Procédé selon l'une des revendications 1 à 8, **caractérisé en ce que** le séchage du sel précipité dans le cristalliseur (8) est effectué sur un filtre-sécheur à bande (12), dont une face est soumise à une pression réduite, la partie du filtre qui reçoit le produit assurant sa séparation primaire par rapport au milieu de précipitation et la partie suivante assurant un séchage par air chaud pulsé à une température inférieure à 50°C pendant un temps compris entre 0,1 et 2h.

10. Procédé selon l'une des revendications 1 à 8, **caractérisé en ce que** le séchage du sel précipité dans le cristalliseur (8) est effectué en envoyant la suspension produite dans le cristalliseur (8), dans un filtre-sécheur rotatif (16) sous pression d'air, l'élément de filtration (20b) étant porté par un organe rotatif (20), qui est entrainé autour de l'axe du stator du filtre-sécheur (16), le liquide filtré à partir de la suspension sortant selon l'axe du stator après avoir traversé l'élément de filtration (20b), l'alimentation en suspension s'effectuant sur un premier secteur du stator, le séchage par soufflage d'air du solide arrêté par l'élément de filtration (20b) étant effectué dans un second secteur du stator atteint par l'organe rotatif (20) après le premier secteur dans le sens de la rotation du rotor, le solide retenu à la surface de l'élément de filtration (20b) étant raclé dans un troisième secteur du stator postérieur au second dans le sens de rotation du rotor et étant évacué en continu hors du stator du filtre-sécheur (16).

## Patentansprüche

1. Verfahren zur Herstellung eines Salzes der *O-*Acetylsalicylsäure mit einer basischen Aminosäure, wobei:
- Zusammenführen, in einem Reaktor (4), zum einen einer wässrigen Lösung einer basischen Aminosäure und zum anderen einer Lösung von O-Acetylsalicylsäure in einem mit Wasser mischbaren organischen Lösungsmittel bei einer Temperatur unter 30 °C unter normalem Druck;
- Gestatten der Durchführung der Reaktion während einer Zeit, die ausreichend ist, um das gewünschte Salz zu erhalten;
- Hinzufügen, in einen Kristallisator (8), zu dem derart gebildeten Reaktionsmilieu bei einer Temperatur unter 40 °C und unter normalem Druck von Aceton, damit das gewünschte Salz ausfällt;
- Isolieren des Produkts von dem erhaltenen flüssigen Milieu und Trocknen des Produkts bei einer Temperatur unter 50 °C;
**dadurch gekennzeichnet, dass** die Aminosäure in den Reaktor in einer Menge eingeführt wird, die derart ist, dass sie in dem Reaktionsmilieu in leichtem Unterschuss in Bezug auf die Stöchiometrie vorhanden ist, wobei die Gewichtskonzentration der Lösungen der zwei Reagenzien unter der Sättigung, aber über 5 Gew.-% für die *O-*Acetylsalicylsäure und 20 Gew.-% für die Aminosäure ist, wobei die Reaktion in Suspension bei einer Temperatur, gewählt zwischen -15 °C und +30 °C in Abhängigkeit von dem verwendeten Lösungsmittel und unter Rühren, stattfindet, und dass das Salz infolge des Hinzufügens von Aceton in den Kristallisator unter energischem Rühren ausfällt, wobei das Salz getrocknet wird, nachdem es von seinem Ausfällungsmilieu getrennt wurde, dass das molare Verhältnis O-Acetylsalicylsäure/basische Aminosäure der in den Reaktor (4) eingeführten Reagenzien zwischen 1:0,97 und 1:0,70, Grenzwerte inklusive, beträgt, dass das Rühren im Kristallisator (8) mit einem Energieaufwand zwischen 0,5 und 2 W je Liter Milieu durchgeführt wird und dass das Verfahren kontinuierlich abläuft.

2. Verfahren nach Anspruch 1, **dadurch gekennzeichnet, dass** die basische Aminosäure das Lysin oder das Arginin oder das Ornithin ist.

3. Verfahren nach Anspruch 2, **dadurch gekennzeichnet, dass** die basische Aminosäure unabhängig von ihrer Form, racemisch oder nicht, monohydratisch oder nicht, das Lysin ist.

4. Verfahren nach einem der Ansprüche 1 bis 3, **dadurch gekennzeichnet, dass** die Reaktionszeit in dem Reaktor (4) unter 1 Stunde beträgt.

5. Verfahren nach einem der Ansprüche 1 bis 4, **dadurch gekennzeichnet, dass** die Temperatur in dem Kristallisator (8) zwischen -15 °C und +15 °C, Grenzwerte inbegriffen, gewählt ist.

6. Verfahren nach einem der Ansprüche 1 bis 5, **dadurch gekennzeichnet, dass** die Zeit zwischen dem Eintritt des Reaktionsmilieus in den Kristallisator (8) und der Isolierung des ausgefällten Salzes in Bezug auf die flüssige Phase des Ausfällungsmilieus unter 1 Stunde beträgt.

7. Verfahren nach einem der Ansprüche 1 bis 6, **dadurch gekennzeichnet, dass** das für das Solubilisieren der O-Acetylsalicylsäure verwendete organischen Lösungsmittel das Ethanol ist und die Reaktion der Säure mit der basischen Aminosäure bei einer Temperatur zwischen -15 °C und 0 °C, Grenzwerte inbegriffen, erfolgt.

8. Verfahren nach einem der Ansprüche 1 bis 6, **dadurch gekennzeichnet, dass** das für das Solubilisieren der O-Acetylsalicylsäure verwendete organischen Lösungsmittel das Aceton ist und die Reaktion der Säure mit der basischen Aminosäure bei einer Temperatur zwischen -5 °C und +30 °C, Grenzwerte inbegriffen, erfolgt.

9. Verfahren nach einem der Ansprüche 1 bis 8, **dadurch gekennzeichnet, dass** das Trocknen des im Kristallisator (8) ausgefällten Salzes auf einem Bandfiltertrockner (12) erfolgt, von dem eine Seite einem reduzierten Druck ausgesetzt ist, wobei der Teil des Filters, der das Produkt empfängt, seine primäre Trennung in Bezug auf das Ausfällungsmilieu gewährleistet und der folgende Teil eine Trocknung durch gepulste Warmluft bei einer Temperatur unter 50 °C während einer Zeit zwischen 0,1 und 2 Stunden inklusive gewährleistet.

10. Verfahren nach einem der Ansprüche 1 bis 8, **dadurch gekennzeichnet, dass** das Trocknen des im Kristallisator (8) ausgefällten Salzes durchgeführt wird, indem die in dem Kristallisator (8) hergestellte Suspension unter Luftdruck in einen Rotationsfiltertrockner (16) geschickt wird, wobei das Filtrationselement (20b) von einem Rotationsorgan (20) getragen wird, das um die Achse des Stators des Filtertrockners (16) angetrieben wird, wobei die auf der Basis der Suspension gefilterte Flüssigkeit nach Durchqueren des Filtrationselements (20b) gemäß der Achse des Stators austritt, wobei die Versorgung mit Suspension über einen ersten Sektor des Stators erfolgt, wobei das Trocknen des vom Filtrationselement (20b) zurückgehaltenen Feststoffs durch Luftblasen in einem zweiten Sektor des Stators erfolgt, der von dem Rotationsorgan (20) nach dem ersten Sektor in Rotationsrichtung des Rotors erreicht wird, wobei der auf der Oberfläche des Filtrationselements (20b) zurückgehaltene Feststoff in einem dritten Sektor nach dem zweiten in Rotationsrichtung des Rotors abgekratzt und kontinuierlich aus dem Stator des Filtertrockners (16) befördert wird.

## Claims

1. A method for preparing a salt of *O-*acetylsalicylic acid of a basic amino acid, wherein:
- an aqueous solution of a basic amino acid, and a solution of O-acetylsalicylic acid in a water-miscible organic solvent, are mixed in a reactor (4) at a temperature below 30°C under atmospheric pressure;
- the reaction is allowed to occur for a sufficient time to obtain the desired salt;
- in a crystalliser (8), acetone is added to the reaction medium thus formed, at a temperature below 40°C and under atmospheric pressure, to precipitate the desired salt;
- the product is isolated from the liquid medium obtained and dried at a temperature below 50°C;
**characterised in that** the amino acid is introduced into the reactor in an amount such that it is present in the reaction medium in slight deficit relative to the stoichiometry, the weight concentration of the solutions of the two reagents being less than saturation but greater than 5% by weight for the *O-*acetylsalicylic acid and 20% by weight for the amino acid, the reaction being carried out in suspension at a temperature chosen between -15°C and +30°C, depending on the solvent used, and with stirring, and that the salt, following the addition of acetone in the crystalliser, precipitates under vigorous stirring, said salt being dried after it has been isolated from its precipitation medium, **in that** the molar ratio of *O-*acetylsalicylic acid to basic amino acid of the reagents introduced into the reactor (4) is between 1:0.97 and 1:0.70, bounds included, **in that** the stirring in the crystalliser (8) is carried out with an energy expenditure of between 0.5 and 2 W per litre of medium and **in that** the method is carried out continuously.

2. The method as claimed in claim 1, **characterised in that** the basic amino acid is lysine or arginine or ornithine.

3. The method as claimed in claim 2, **characterised in that** the basic amino acid is lysine whatever its form, racemic or not, monohydrate or not.

4. The method as claimed in one of claims 1 to 3, **characterised in that** the reaction time in the reactor (4) is less than 1 hour.

5. The method as claimed in one of claims 1 to 4, **characterised in that** the temperature in the crystalliser (8) is chosen between -15°C and +15°C, bounds included.

6. The method as claimed in one of claims 1 to 5, **characterised in that** the period between the entry of the reaction medium into the crystalliser (8) and the isolation of the precipitated salt from the liquid phase of the precipitation medium is less than 1 hour.

7. The method as claimed in one of claims 1 to 6, **characterised in that** the organic solvent used for the solution of O-acetylsalicylic acid is ethanol and the reaction of said acid with the basic amino acid is carried out at a temperature between -15°C and 0°C, bounds included.

8. The method as claimed in one of claims 1 to 6, **characterised in that** the organic solvent used for the solution of O-acetylsalicylic acid is acetone and the reaction of said acid with the basic amino acid is carried out at a temperature between -5°C and +30°C, bounds included.

9. The method as claimed in one of claims 1 to 8, **characterised in that** the drying of the salt precipitated in the crystalliser (8) is carried out on a belt filter-dryer (12), one side of which is subjected to a reduced pressure, the part of the filter which receives the product ensuring its primary separation from the precipitation medium and the following part ensuring drying by forced hot air at a temperature below 50°C for a period between 0.1 and 2 h.

10. The method as claimed in one of claims 1 to 8, **characterised in that** the drying of the salt precipitated in the crystalliser (8) is carried out by sending the suspension produced in the crystalliser (8) into a rotary filter-drier (16) under air pressure, the filter element (20b) being carried by a rotating member (20), which is driven about the axis of the stator of the filter-drier (16), the liquid filtered from the suspension leaving along the axis of the stator after passing through the filter element (20b), the suspension feed being carried out on a first sector of the stator, the air-blow drying of the solid stopped by the filter element (20b) being carried out in a second sector of the stator reached by the rotating member (20) after the first sector in the direction of rotation of the rotor, the solid retained on the surface of the filter element (20b) being scraped off in a third sector of the stator after the second in the direction of rotation of the rotor and being continuously discharged from the stator of the filter-dryer (16).
